# EUROPÄISCHE PATENTSCHRIFT

(11) **EP 1 238 276 B1**
(45) Veröffentlichungstag und Bekanntmachung des Hinweises auf die Patenterteilung: **03.03.2004**
(21) Anmeldenummer: 00983230.4
(22) Anmeldetag: 06.12.2000
(51) Int. Cl.: G01N 33/50, G01N 33/543, A61K 7/06

(54) **VERFAHREN ZUR BESTIMMUNG DES SCHÄDIGUNGSGRADES VON KERATINISCHEN FASERN (HAARE)**
PROCESS FOR DETERMINING THE DEGENERATION LEVEL OF KERATINIC FIBERS (HAIR)
PROCEDE SERVANT A DETERMINER LE NIVEAU DE DEGRADATION DE FIBRES KERATINIQUES (POILS)

(30) Priorität: 08.12.1999 DE 19959213
(43) Veröffentlichungstag der Anmeldung: 11.09.2002
(73) Patentinhaber: Henkel Kommanditgesellschaft auf Aktien, 40589 Düsseldorf (DE)
(72) Erfinder: SCHULZE ZUR WIESCHE, Erik, 40219 Düsseldorf (DE); BROCKMANN, Claudia, 40627 Düsseldorf (DE); HOLLENBERG, Detlef, 40699 Erkrath (DE)
(86) Internationale Anmeldenummer: PCT/EP2000/012265
(87) Internationale Veröffentlichungsnummer: WO 2001/042783

(56) Entgegenhaltungen:
- EP-A- 0 148 994
- DE-C- 4 432 854
- FR-A- 2 473 284
- US-A- 5 131 417
- DATABASE WPI Section Ch, Week 199638 Derwent Publications Ltd., London, GB; Class D21, AN 1996-374941 XP002164288 & JP 08 178920 A (KANEBO LTD), 12. Juli 1996 (1996-07-12)
- PATENT ABSTRACTS OF JAPAN vol. 012, no. 429 (P-785), 14. November 1988 (1988-11-14) & JP 63 163143 A (NEC CORP), 6. Juli 1988 (1988-07-06)
- PATENT ABSTRACTS OF JAPAN vol. 1996, no. 08, 30. August 1996 (1996-08-30) & JP 08 101193 A (KAO CORP), 16. April 1996 (1996-04-16)
- PATENT ABSTRACTS OF JAPAN vol. 018, no. 670 (P-1845), 16. Dezember 1994 (1994-12-16) & JP 06 265544 A (KANEBO LTD), 22. September 1994 (1994-09-22)

## Beschreibung

Die Erfindung betrifft ein Verfahren zur Bestimmung von Schädigungen an keratinischen Fasern.

Keratinische Fasern, sei es als Werkstoffe wie Wolle oder als Teil biologischer Systeme wie menschliche Haare, sind einer Reihe von Umwelteinflüssen ausgesetzt, die zu mehr oder weniger großen Schädigungen der Fasern führen können. Im Falle menschlicher Haare können dies natürliche Einflüsse wie starke Sonnenbestrahlung sein. Es kommen aber auch künstliche Einflüsse wie Fönen, Kämmen oder Bürsten sowie Blondieren, Dauerwellen oder Färben in Betracht, die bei zu häufiger, zu starker oder unsachgemäßer Durchführung auch zu Schädigungen des Haares führen können.

Für den Erfolg bestimmter Haarbehandlungen, wie insbesondere Dauerwellen und Färben, kann es aber von enormen Vorteil sein, wenn der die Behandlung durchführende Friseur Kenntnis vom Schädigungsgrad des Haares hat. Es kann dann seine Behandlung mit Hilfe seines Fachwissens auf den jeweiligen Schädigungsgrad des Haares abstimmen oder gegebenenfalls eine repair-Behandlung vorschalten. Für ein einheitliches Ergebnis einer Färbebehandlung kann es weiterhin entscheidend sein. ob einheitlich geschädigtes oder ungeschädigtes Haar vorliegt oder ob stark unterschiedlich geschädigte Haarbereiche, z.B. praktisch ungeschädigtes Haar im Ansatz und stark geschädigtes Haar in den Spitzen, vorliegt.

Die Bestimmung des Schädigungsgrades von Haaren ist aber bei den bekannten Verfahren mit einem relativ hohen Aufwand verbunden und erfordert zudem eine hohe Erfahrung und gute Fachkenntnisse von der die Bestimmung durchführenden Person.

Es besteht daher ein Bedürfnis nach einem einfachen Verfahren, das es erlaubt, mit einfachen Mitteln und geringem Zeitaufwand den Schädigungsgrad von keratinischen Fasern zumindest qualitativ oder halbquantitativ zu bestimmen. Dieses Verfahren sollte es dem Friseur ermöglichen, den individuellen Schädigungsgrad der Haare unmittelbar vor der Behandlung auf schnelle und einfache Weise gleichwohl aber mit sicherem Ergebnis zu ermitteln. Idealerweise sollte dieses Verfahren aber auch dem über keinerlei einschlägige Fachkenntnis oder Erfahrungen verfügenden Konsumenten erlauben, aus der immer größer werdenden Zahl an Varianten der modernen Haarpflege- und Behandlungsserien das für den Schädigungsgrad seiner Haare optimale Produkt auszuwählen.

Es wurde nun überraschenderweise gefunden, daß mit Hilfe eines Mittels, das zwei nicht mischbare kontinuierliche flüssige Phasen unterschiedlicher Dichte und Polarität aufweist, auf einfache und schnelle Weise mit hoher Reproduzierbarkeit Aussagen über den Schädigungsgrad keratinischer Fasern erhalten werden können.

Gegenstand der Erfindung ist daher ein Verfahren zur Bestimmung des Schädigungsgrades von keratinischen Fasern, insbesondere menschlichen Haaren, dadurch gekennzeichnet, daß man die Fasern in einen Behälter einbringt, der ein flüssiges Mittel enthält, das mindestens zwei miteinander nicht mischbare kontinuierliche flüssige Phasen A, B und gegebenenfalls C aufweist, deren Dichte sich bei 20 °C jeweils um mindestens 0,04 g/ml unterscheidet, und auf das System einen Impuls ausübt.

Unter keratinischen Fasern werden erfindungsgemäß Wolle, Pelze und Federn, insbesondere aber menschliche Haare verstanden.

Dem erfindungsgemäßen Verfahren liegen Beobachtungen beim Einbringen von Haaren in ein System zweier nichtmischbarer Flüssigkeiten unterschiedlicher Polarität zugrunde. Bei Einhaltung bestimmter Bedingungen, die im weiteren noch ausführlich beschrieben werden, sammeln sich ungeschädigte oder wenig geschädigte Haare bevorzugt in der apolaren Phase und stark geschädigte Haare bevorzugt in der polaren Phase an.

Gemäß einer bevorzugten Ausführungsform weist das erfindungsgemäße flüssige Mittel zwei miteinander nicht mischbare kontinuierliche Phasen A und B auf. Die flüssige Phase mit der größeren Polarität wird im weiteren als Phase A bezeichnet.

Die Definition der "Polarität" der Phase bezieht sich im Rahmen dieser Anmeldung auf die Dipolmomente der oder des die Phase bildenden Stoffe oder Stoffes. Bevorzugt wird die Phase A im wesentlichen, d.h. zu 90 Gew.-% und mehr, bezogen auf die Phase A, von Substanzen gebildet, die ein Dipolmoment von 1,0 Debye und größer aufweisen.

Gemäß einer ersten bevorzugten Ausführungsform besteht die Phase A ausschließlich oder zu mehr als 90 Gew.-%, bezogen auf die Phase A, aus Wasser. Als weitere Komponenten kommen bevorzugt Tenside, Farbstoffe und anorganische oder organische Salze sowie Parfümöle in Betracht.

Als Tenside können anionischen, ampholytische, zwitterionischen, nichtionogenen und kationische Tenside eingesetzt werden.

Als anionische Tenside eignen sich für die Durchführung des erfindungsgemäßen Verfahrens alle bekannten anionischen oberflächenaktiven Stoffe. Diese sind gekennzeichnet durch eine wasserlöslich machende, anionische Gruppe wie z. B. eine Carboxylat-, Sulfat-. Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium-, Magnesium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Amidethercarboxylate der Formel [R-NH(-CH₂-CH₂-O)ₙ-CH₂-COO]ₘZ, in der R für einen linearen oder verzweigten, gesättigten oder ungesättigten Acylrest mit 2 bis 29 C-Atomen, n für ganze Zahlen von 1 bis 10, m für die Zahlen 1 oder 2 und Z für ein Kation aus der Gruppe der Alkali- oder Erdalkalimetalle steht,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(-CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Kokosmonoglyceridsulfate.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel RO-(S)ₓ. Diese Verbindungen sind durch die folgenden Parameter gekennzeichnet.

Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein S können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" I Iomologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können zwitterionische Tenside verwendet werden. Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weiterhin können die sehr gut biologisch abbaubaren quaternären Esterverbindungen, wie beispielsweise die unter dem Warenzeichen Stepantex® vertriebenen Dialkylammoniummethosulfate und Methyl-hydroxyalkyl-dialkoyloxyalkyl-ammoniummethosulfate und die entsprechenden Produkte der Dehyquart®-Reihe, als kationische Tenside eingesetzt werden. Weitere erfindungsgemäß verwendbare kationische Tenside stellen die quaternisierten Proteinhydrolysate dar.

Als anorganische Salze können in der Phase A beispielsweise die Halogenide, insbesondere Chloride, Sulfate, Phosphate und Carbonate von Alkalimetallen, insbesondere Natrium und Kalium, Erdalkalimetallen, insbesondere Magnesium, Mangan, Zink, Eisen, Kupfer und Aluminium enthalten sein. Als organische Salze kommen insbesondere die Acetate, Tartrate und Citrate der oben genannten Metalle sowie die entsprechenden Ammonium- und Alkanolammoniumsalze in Betracht.

Als Farbstoffe werden bevorzugt die auch in Kosmetika, z.B. zum Anfärben von Shampoos, eingesetzten Farbstoffe verwendet.

Als Parfümöle können solche mit der Duftnote einer Frucht, wie beispielsweise von Apfel, Birne, Erdbeere, Pfirsich, Aprikose, Ananas, Banane, Kirsche, Kiwi, Mango, Kokos, Mandel, Grapefruit, Maracuja, Mandarine und Melone, bevorzugt sein.

Die Phase B besteht im wesentlichen, d. h. zu mehr als 90 Gew.-%, bezogen auf die Phase B, aus apolaren Substanzen.

Als Grundbestandteile der Phase B eignen sich prinzipiell alle wasserunlöslichen Öle und Fettstoffe. Als wasserunlöslich werden erfindungsgemäß solche Stoffe definiert, deren Löslichkeit in Wasser bei 20 °C kleiner als 0,1 Gew.-% beträgt. Der Schmelzpunkt der einzelnen Öl- oder Fettkomponenten sollte möglichst unterhalb von 5 °C liegen. Bei Verwendung mehrerer Öl- und Fettkomponenten sowie ggf. festen Paraffinen und Wachsen ist es in der Regel jedoch auch ausreichend, wenn die Mischung der Öl- und Fettkomponenten diesen Bedingungen genugt.

Solche Ölkomponenten sind beispielsweise Kohlenwasserstoffe, Silikonöle, Pflanzenöle sowie Fettsäuren und Fettsäure-Derivate. Diese Substanzen sollten selbst oder im Rahmen der gewählten Mischung zumindest in einem Temperaturbereich von ca. 5 °C bis ca. 30 °C flüssig sein.

Als Kohlenwasserstoffe können lineare und verzweigte, gesättigte und ein- oder mehrfach ungesättigte Kohlenwasserstoffe eingesetzt werden. Bevorzugt sind die kommerziell erhältlichen Paraffinöle sowie synthetische Kohlenwasserstoffe und Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-isopentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.

Ebenfalls erfindungsgemäß als Ölkomponenten einsetzbar sind Fettsäure- und Fettalkoholester. Bevorzugt sind die Monoester der Fettsäuren mit Alkoholen mit 3 bis 24 C-Atomen. Bei dieser Stoffgruppe handelt es sich um die Produkte der Veresterung von Fettsäuren mit 6 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Oxidation von Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat, Isononansäure-C16-18-alkylester (Cetiol® SN), Stearinsäure-2-ethylhexylester (Cetiol® 868), Cetyloleat, Glycerintricaprylat, Kokosfettalkohol-caprinat/caprylat und n-Butylstearat.

Schließlich stellen auch Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-diisostearat und Neopentylglykoldicapylat erfindungsgemäß verwendbare Ölkomponenten dar.

Ebenfalls als Ölkomponente einsetzbar sind Fettalkohole mit 8 bis 22 C-Atomen. Die eingesetzten Fettalkohole können gesättigt oder ungesättigt und linear oder verzweigt sein. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkuhol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen.

Ester, Ether, Amide und Fettalkohole sind erfindungsgemäß bevorzugte Fettsäurederivate.

Als Silikonöle erfindungsgemäß einsetzbar sind insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie das Handelsprodukt Fancorsil® LIM-1.

Bevorzugte pflanzliche Öle sind Sonnenblumenöl, Orangenöl, Mandelöl, Weizenkeimöl und Pfirsichkernöl.

Neben den genannten apolaren Verbindungen kann die Phase B weiterhin bevorzugt noch öllösliche oberflächenaktive Verbindungen, Farbstoffe und Parfümöle enthalten.

Beispiele für bevorzugte Zweiphasensysteme sind die folgenden Systeme, bei denen die einzelnen Phasen jeweils bevorzugt zu 90 und mehr Gew.-% aus der angegebenen Haupt-Komponente bestehen:
- (A): demineralisiertes Wasser / (B): Isododecan
- (A): Dimethicon / (B): Isododecan

Gemäß einer bevorzugten Ausführungsform enthält zumindest die Phase A oder die Phase B einen Farbstoff. Der Einsatz unterschiedlicher Farbstoffe in den Phasen A und B kann besonders bevorzugt sein.

Es hat sich als vorteilhaft für das erfindungsgemäße Verfahren herausgestellt, wenn sich die kontinuierlichen Phasen A und B bei 20 °C in ihren Dichten um mindestens 0,1, insbesondere um mindestens 0,2 g/ml unterscheiden.

Das Verfahren kann prinzipiell in Behältern aus einem beliebigen, vorteilhafterweise durchsichtigem, Material durchgeführt werden. Es hat sich aber als vorteilhaft herausgestellt, wenn der Behälter vollständig oder zumindest zu großen Teilen aus Glas besteht. Ein Beispiel für einen solchen Behälter sind Schraubdeckelgläser mit einem Plastikverschluß. Der Formgestaltung der Behälter sind aber keine prinzipielle Grenzen gesetzt. So kann sich die Form des Behälters beispielsweise aus ästhetischen Gründen an den Behältern orientieren, wie sie beispielsweise im Souvenier-Bereich für sogenannte "Schneelandschaften" gewählt werden.

Zur Durchführung des Verfahrens positioniert man die Haarproben in dem Behälter mit den beiden Phasen A und B so, daß sie sich zunächst auf der Oberfläche der oberen Phase befinden.

Als Haarproben wählt man 5-1000 vorteilhafterweise etwa 20-100, jeweils ca. 0,5 - 1 cm lange Haarabschnitte möglichst gleicher Länge. Es hat sich als vorteilhaft wenngleich nicht als unbedingt notwendig erwiesen, wenn die Haare vor dem Abschneiden der Haarabschnitte shampooniert und getrocknet werden.

Die Dimensionen des Behälters und die Mengen der Phasen A und B werden vorteilhafterweise so gewählt, daß ca. 5 - 40 Haare pro cm² Grenzfläche zwischen den Phasen A und B zur Untersuchung kommen. Weiterhin sollte die Phase mit der geringeren Dichte, in den meisten Fällen die Phase B, im Behälter eine Höhe aufweisen, die kleiner als die Länge der Haarabschnitte ist. Die Höhe der Phase mit der größeren Dichte sollte zur guten Sichtbarkeit des Effektes etwa 2 bis etwa 10 mal so groß wie die Höhe der Phase mit der geringeren Dichte sein.

Oberhalb der beiden flüssigen Phasen kann sich im Behälter noch eine Luftschicht befinden; es ist aber auch möglich, den Behälter in seinen Dimensionen so zu wählen, daß nach dem Verschließen praktisch kein Raum mehr für eine Luftphase verbleibt.

Vor der Ausübung des Impulses wird der Behälter mit den beiden Phasen und der Haarprobe verschlossen. Für die Ausübung des Impulses haben sich zwei Vorgehensweisen als besonders geeignet erwiesen:
1. Der Behälter wird für ca. 1 Sekunde auf den Kopf gestellt. Danach wird er wieder in die ursprüngliche Lage verbracht. Insgesamt wird also eine Drehung um 360 ° um eine horizontale Achse durchgeführt.
2. Der Behälter wird für eine Zeit von ca 10 bis 30 Sekunden vorsichtig geschüttelt. Unter vorsichtigem Schütteln wird eine solche Schüttelbewegung verstanden, die Blasenbildung so weit als möglich vermeidet.

Je stärker die Haarproben geschädigt sind, umso schneller und vollständiger bewegen sie sich in die polare Phase. Bei der bevorzugten Anordnung (Phase A weist eine höhere Dichte als Phase B auf) werden geschädigte Haare also auf den Boden des Behälters absinken. Ungeschädigte oder nur leicht geschädigte Haare werden dagegen auf oder in der oberen Schicht verbleiben.

Wenngleich, wie oben beschrieben, flüssige Mittel bevorzugt sind, die zwei miteinander nicht mischbare kontinuierliche flüssige Phasen A und B aufweisen, so können in einzelnen Fällen flüssige Mittel, die drei miteinander nicht mischbare kontinuierliche flüssige Phasen A, B und C aufweisen, Vorteile hinsichtlich einer schnellen und genauen Ablesbarkeit der Ergebnisse bieten. Beispiele für solche Dreiphasensysteme sind die folgenden Systeme, bei denen die einzelnen Phasen jeweils bevorzugt zu 90 und mehr Gew.-% aus der angegebenen Haupt-Komponente bestehen:
- (A): demineralisiertes Wasser / (B): Isododecan / (C): Perfluordecalin
- (A): demineralisiertes Wasser / (B): Isododecan / (C): Dimethicon
- (A): demineralisiertes Wasser / (B): 1,3-Bis(2-Ethylhexyl)-cyclohexan / (C): Propylen-carbonat

Die folgenden Beispiele sollen die Erfindung näher erläutern.

### Beispiele:

### Beispiel 1

Es wurden 2 Proben zu je ca. 50 Haarabschnitten einer Länge von 0,7 cm von einer Probe gesunden kaukasischen Menschenhaares abgeschnitten.

Die erste Probe wurde mit einem handelsüblichen Shampoo gewaschen und anschließend an der Luft getrocknet.

Die zweite Probe wurde zweimal im Abstand einer Woche mit einem handelsüblichen Blondiermittel ultrablondiert, dann mit einem handelsüblichen Shampoo gewaschen und an der Luft getrocknet.

Anschließend wurden die Proben in ein handelsübliches Weithals-Schraubdeckelglas mit einem Volumen von 30 ml eingebracht. Dieses Glas enthielt
- als Phase A:
   23,0 ml demineralisiertes Wasser
   3 Tropfen Farbstofflösung Kelate Cu (EDTA-Kupfer-Dinatriumsalz (Tri-K))
- als Phase B:
   2,0 ml Isododecan
   1 Tropfen Farbstoff Solvent Blue 35 (Fa. Ellis & Everard)

Die Haare wurden vorsichtig auf die Oberfläche der oberen Flüssigkeit gegeben. Danach wurde das Glas zugeschraubt und 30 Sekunden lang, wie oben beschrieben, vorsichtig geschüttelt.

Bei der Probe mit nicht vorbehandelten gesunden Haaren verblieben alle Haare in der oberen Phase (Phase B).

Bei der Probe mit zweifach ultrablondierten Haaren fanden sich alle Haare auf dem Boden des Glases bzw. in der unteren Phase (Phase A) wieder.

### Beispiel 2

Es wurden 10 mal jeweils 10 Haarabschnitte gemäß Beispiel 1 unterschiedlichen haarverändernden Behandlungen unterworfen.

Danach wurde der Schädigungsgrad mit dem erfindungsgemäßen Testverfahren, wie in Beispiel 1 beschrieben, bestimmt.

Anschließend wurden die Haarabschnitte einer Aminosäureanalyse nach dem Verfahren unterworfen, das in der Druckschrift H.D. Spackman, W.H. Stein, S. Moore, Anal. Chem. (1958), 1190-1206 beschrieben ist. Diese Aminosäureanalyse ist eine etablierte Methode zur Charakterisierung von Schädigungen bei Humanhaaren und Wolle. Je höher der Anteil an Cysteinsäure ist, umso größer ist der Schädigungsgrad des Haares bzw. der Wolle.

Die Ergebnisse, die mit diesen beiden Bestimmungsmethoden erzielt wurden, sind in der folgenden Tabelle zusammengefaßt:

| Vorbehandlung der Haare | Aminosäureanalyse [mol-% Cysteinsäure] | erfindungsgemäßes Verfahren [abgesunkene Haare (in unterer Phase bzw. auf dem Boden) in %] |
|---|---|---|
| unbehandelt | 1,8 | 0 |
| 1 x gebleicht | 2,4 | 27 |
| 1 x gebleicht + 1 x dauergewellt | 3,7 | 49 |
| 2 x gebleicht + 2 x dauergewellt | 7,0 | 62 |

## Patentansprüche

1. Verfahren zur Bestimmung des Schädigungsgrades von keratinischen Fasern, insbesondere menschlichen Haaren, **dadurch gekennzeichnet, daß** man die Fasern in einen Behälter einbringt, der ein flüssiges Mittel enthält, das mindestens zwei miteinander nicht mischbare kontinuierliche flüssige Phasen aufweist, deren Dichte sich jeweils bei 20 °C um mindestens 0,04 g/ml unterscheidet, und auf das System einen Impuls ausübt.

2. Verfahren nach Anspruch 1, **dadurch gekennzeichnet, daß** das flüssige Mittel zwei miteinander nicht mischbare kontinuierliche Phasen A und B aufweist.

3. Verfahren nach Anspruch 1 oder 2, **dadurch gekennzeichnet, daß** die kontinuierliche Phase A im wesentlichen aus Wasser besteht.

4. Verfahren nach einem der Ansprüche 1 bis 3, **dadurch gekennzeichnet, daß** die kontinuierliche Phase B im wesentlichen aus apolaren Verbindungen besteht, die ausgewählt sind aus Kohlenwasserstoffen, Silikonölen, Pflanzenölen sowie Fettsäuren und Fettsäure-Derivaten.

5. Verfahren nach Anspruch 4, **dadurch gekennzeichnet, daß** die Fettsäure-Derivate ausgewählt sind aus Estern, Ethern, Amiden und Fettalkoholen.

6. Verfahren nach einem der Ansprüche 2 bis 5, **dadurch gekennzeichnet, daß** sich die kontinuierlichen Phasen A und B bei 20 °C in ihren Dichten um mindestens 0,1, insbesondere um mindestens 0,2 g/ml unterscheiden.

7. Verfahren nach einem der Ansprüche 1 bis 6, **dadurch gekennzeichnet, daß** der Behälter aus Glas besteht.

## Claims

1. A process for determining the degree of damage to keratinous fibers, more particularly human hair, **characterized in that** the fibers are introduced into a container holding a liquid preparation comprising at least two immiscible continuous liquid phases which differ in their densities at 20°C by at least 0.04 g/ml, and an impulse is applied to the system.

2. A process as claimed in claim 1, **characterized in that** the liquid preparation comprises two immiscible continuous phases A and B.

3. A process as claimed in claim 1 or 2, **characterized in that** the continuous phase A consists essentially of water.

4. A process as claimed in any of claims 1 to 3, **characterized in that** the continuous phase B consists essentially of nonpolar compounds selected from hydrocarbons, silicone oils, vegetable oils and fatty acids and fatty acid derivatives.

5. A process as claimed in claim 4, **characterized in that** the fatty acid derivatives are selected from esters, ethers, amides and fatty alcohols.

6. A process as claimed in any of claims 2 to 5, **characterized in that** the continuous phases A and B differ in their densities at 20°C by at least 0.1 and more particularly by at least 0.2 g/ml.

7. A process as claimed in any of claims 1 to 6, **characterized in that** the container consists of glass.

## Revendications

1. Procédé pour la détermination du degré de dégradation de fibres kératiniques, en particulier de cheveux humains, **caractérisé en ce qu'**on introduit les fibres dans un récipient qui contient un agent liquide qui présente au moins deux phases liquides continues non miscibles l'une à l'autre, dont la densité se distingue, respectivement à 20 °C, à concurrence d'au moins 0,04 g/ml, et **en ce qu'**on exerce une impulsion sur le système.

2. Procédé selon la revendication 1, **caractérisé en ce que** l'agent liquide présente deux phases continues A et B non miscibles l'une à l'autre.

3. Procédé selon la revendication 1 ou 2, **caractérisé en ce que** la phase continue A est constituée essentiellement d'eau.

4. Procédé selon l'une quelconque des revendications 1 à 3, **caractérisé en ce que** la phase continue B est constituée essentiellement par des composés apolaires qui sont choisis parmi le groupe comprenant des hydrocarbures, des huiles de silicone, des huiles végétales ainsi que des acides gras et des dérivés d'acides gras.

5. Procédé selon la revendication 4, **caractérisé en ce que** les dérivés d'acides gras sont choisis parmi le groupe comprenant des esters, des éthers, des amides et des alcools gras.

6. Procédé selon l'une quelconque des revendications 2 à 5, **caractérisé en ce que** les phases continues A et B se distinguent, à 20 °C, en ce qui concerne leurs densités, à concurrence d'au moins 0,1, en particulier à concurrence d'au moins 0,2 g/ml.

7. Procédé selon l'une quelconque des revendications 1 à 6, **caractérisé en ce que** le récipient est constitué de verre.
